# EUROPEAN PATENT APPLICATION

(11) **EP 3 903 722 A1**
(43) Date of publication of application: **03.11.2021**
(21) Application number: 21171003.3
(22) Date of filing: 28.04.2021
(51) Int. Cl.: A61B 90/40, A61G 10/00

(54) **PATHOGEN CONTAINMENT DEVICE**

(30) Priority: 01.05.2020 US 202063019244 P
(71) Applicant: Majzoub, Ibrahim, Oak Brook, Illinois 60523 (US); Agha, Rania, Oak Brook, Il 60523 (US)
(72) Inventor: Majzoub, Ibrahim, Oak Brook, Illinois 60523 (US); Agha, Rania, Oak Brook, Il 60523 (US)
(74) Representative: Elkington and Fife LLP

(57) **Abstract**

The present invention provides a safer way to intubate patients having communicable diseases, such as bacterial and viral infections. It protects health care professionals (9) performing the intubation from bodily fluids of the patients that might otherwise be projected directly onto them by coughing, gagging, vomiting, or sneezing. Additionally, it traps airborne suspensions or aerosols containing pathogens, such as bacterial, myco-bacterial, viral, or fungal infections, originating from the patient (7) and keeps them from entering into the air in close proximity to the healthcare professionals. The contaminated air can then be vented to a location which is away from personnel, such as an outdoor containment area. In one embodiment of this invention the contaminated air is passed through a disinfecting solution which destroys the pathogens before the air is recycled into the environment, such as the room where the intubation is carried out.

## Description

### Background of the Invention

Endotracheal intubation involves passing a tube through the mouth/nose, between the vocal cords, and ultimately into the trachea of a patient. Intubation is typically carried out for the purpose of helping a patient to breathe during surgical procedures, after an injury, and to facilitate breathing which is compromised by virtue of complications resulting from heart failure, collapsed lungs, cancer, bacterial infections (such as pneumonia, chronic obstructive pulmonary disease, or severe asthma), and viral infections. For example, thousands of patients are currently being intubated for the purpose of being mechanically ventilated as a result of severe cases of coronavirus (COVID-19). Thousands of other patients having a wide variety of other infectious diseases are also being intubated for one purpose or another.

In any case, coronavirus is a highly infectious disease which poses a great risk to healthcare workers that are caring for patients that are infected with this dangerous virus. Intubation of such patients is particularly dangerous because during the intubation procedure patients exhale virus laden breath and also frequently cough, gag, vomit, sneeze, and otherwise project bodily fluids and pathogen containing aerosols into the atmosphere in close proximity to or even onto the health care workers that are intubating the patient. The incidence of being infected with coronavirus among such health care workers is alarmingly high even though such health care workers wear masks, shields, goggles, gowns, and other protective gear. Accordingly, there is a critical need for such health care professionals to be further protected from pathogens, such as COVID-19, while intubating patients.

Aerosol boxes which are designed to cover the head of a patient during an intubation procedure are used in some cases. Such aerosol boxes are comprised of a clear plastic which allow the healthcare professional with an unobstructed view of the patient to facilitate carrying out the intubation procedure. Such prior art devices typically include two openings situated on the back wall of the aerosol box to allow for the healthcare professional administering the intubation to have access of his or her hands to the head and mouth of the patient. Such prior art device will inherently block bodily fluids from being directly projected onto healthcare workers that are administering the intubation. However, it does not prevent airborne suspensions or aerosols containing pathogens from escaping from the box and entering into the air in close proximity to the healthcare professionals. Another problem with conventional aerosol boxes of the prior art is that they required a great deal of storage space. This can lead to difficulties in the case health care facilities which have only a limited amount of storage space in supply rooms and storage areas. Ridged aerosol boxes are also costly and difficult to transport in large numbers by virtue of being bulky and in some cases susceptible to being damaged. In the case of reusable aerosol boxes cleaning and sterilization between patients is also difficult and time consuming. Rigid aerosol boxes also present a solid waste disposal problem by virtue of being bulky and potentially contaminated with dangerous pathogens.

Better pathogen containment systems are needed to better protect doctors, nurses, and other healthcare professionals from airborne pathogens, such as bacterial and virus, during the intubation of patients and in carrying out other procedures. There is also a continuing need for a pathogen containment system than can be transported and stored in a compacted state and which can be easily a quickly assembled for use. Preferably such a pathogen containment system is low enough in cost to make it commercially viable to be used as a single use disposable product. After being used it should also be capable of being compacted again for easy of disposal as solid waste.

### Summary of the Invention

The present invention provides a safer way to intubate and to carry out other procedures on patients having communicable diseases, such as bacterial and viral infections. It protects health care professionals performing the intubation from bodily fluids of the patients that might otherwise be projected directly onto them by coughing, gagging, vomiting, or sneezing. Additionally, it traps airborne suspensions or aerosols containing pathogens originating from the patient and keeps them entering into the air in close proximity to the healthcare professionals. The contaminated air can then be vented to a location which is away from personnel, such as an outdoor containment area. In one embodiment of this invention the contaminated air is passed through a disinfecting solution which destroys the pathogens before the air is recycled into the environment, such as the room where the intubation is carried out. In still another embodiment of the invention the contaminated air from the device is filtered to remove pathogens. Passing the exhaust gas from the device of this invention through a disinfecting solution and/or filtering it is of particular value in cases where it is not possible or practical to vent the contaminated air to a safe location.

In one highly preferred embodiment of this invention the pathogen containments device is provided in a configuration that allows it to be shipped in stored in a highly compact form. In this embodiment of the invention the side walls and back wall of the pathogen containment chamber are designed so that they can be folded together into a compact form which can nest into the top of the containment chamber. This allows for easy shipment and storage in packaging of minimal volume. Additionally, the pathogen containment chamber can be easily and quickly assembled by simply unfolding the sides from the back (the sides will be extended at an angel of approximately 90° from the back). Then, the assembly of the pathogen containment chamber is completed by simply placing the top on the extended sides and back of the chamber. The pathogen containment system of this invention is preferably made of a low cost polyester, such as polyethylene terephthalate, with will provide it with adequate strength, durability, and clarity for its intended used at a low enough in cost to make it commercially viable to be used as a single use disposable product. In this embodiment of the invention after being used the pathogen containment chamber can also be again folded into a compacted form to allow for easy disposal which occupies minimal volume as solid waste.

The subject invention more specifically discloses a pathogen containment device which is particularly useful for intubating human patients, said device being comprised of a containment chamber which is adapted for being positioned over and covering the head of the patient, said containment chamber having two access ports which are adapted for allowing the hands of a health care worker access into the containment chamber, and said containment chamber having an air exhaust coupling, wherein the containment chamber is at least partially comprised of an essentially transparent material which allows for the health care worker to have a sufficient view of the head of the patient to perform intubation of the patient.

The present invention further reveals a pathogen containment kit which is comprised of a pathogen containment device which is particularly useful for intubating human patients, said device being comprised of a containment chamber which is adapted for being positioned over and covering the head of the patient, said containment chamber having two access ports which are adapted for allowing the hands of a health care worker access into the containment chamber, and said containment chamber having an air exhaust coupling, wherein the containment chamber is at least partially comprised of an essentially transparent material which allows for the health care worker to have a sufficient view of the head of the patient to perform intubation of the patient, and wherein the sides of the containment chamber are folded together and enclosed within a sealed bag.

The present invention also discloses a method for intubating a patient which comprises (1) positioning the patient in an essentially horizontal position for performing the intubation; (2) positioning a pathogen containment device over the head of the patient, wherein the pathogen containment device is comprised of a containment chamber which is adapted for being positioned over and covering the head of the patient, said containment chamber having two access ports which are adapted for allowing the hands of a health care worker access into the containment chamber, and said containment chamber having an air exhaust coupling and a means for exhausting air from the containment chamber through the air exhaust coupling, wherein the containment chamber is at least partially comprised of an essentially transparent material which allows for the health care worker to have a sufficient view of the head of the patient to perform intubation of the patient; (3) activating the means for exhausting air from the containment chamber; and (4) intubating the patient. In most cases it is desirable to keep the pathogen containment device positioned over the head of the patient after completion of the intubation procedure and throughout the entire period during which the patient is contagious. In such cases, it is advantageous to seal all access ports to reduce the risk of pathogens escaping and to minimize the required level of exhaust air flow. For instance, during the intubation procedure it may be desirable to exhaust air from the pathogen containment chamber at a flow rate of 1500 to 3000 liters per minute. However, after completing the procedure and plugging the access ports an air flow art of only 30 to 50 liters per minute may be required. In such procedures the access ports can easily be plugged by simply affixing a plastic film over them by use of tape or any other suitable means.

### Brief Description of the Drawings

Fig-1 is a front side view of the pathogen containment device of this invention with the head of a patient shown within the containment chamber of the device.
Fig-2 is a front view of the containment chamber showing the hands of a healthcare professional extending through the access ports on the back wall of the containment chamber as a patient is being intubated.
Fig-3 illustrates the hands of a healthcare professional extending into the interior of the pathogen containment chamber of the pathogen containment device of this invention with a laryngoscope.
Fig-4 illustrates a patient in the pathogen containment device of this invention wherein the body of the patient is substantially covered with a flexible sheet that extends from the pathogen containment chamber of the device.
Fig-5 is a top view of the pathogen containment device showing an optional entry port and a roll of flexible sheet which is attached to the containment chamber and which can be extended partially or totally over a patient in a manner that impedes pathogens from escaping from the containment chamber.
Fig-6 is a front perspective view of the pathogen containment device showing an optional entry port and a flexible sheet which is attached to the containment chamber and which substantially covers the patient in a manner that impedes pathogens from escaping from the containment chamber.
Fig-7 is a side view of the pathogen containment chamber with an air exhaust line connected to the air exhaust coupling on the side of the chamber.
Fig-8 is an exploded view of a pathogen containment kit of this invention.
Fig-9 illustrates the wall assembly of the pathogen containment chamber with the left side wall and the right side wall being fully extended from the back wall.
Fig-10 is an exploded view showing the left side wall and the right side wall extending from the back wall at 90° angles which allows the top to be lowered firmly onto the wall assembly so as to make an essentially air tight seal between the left side wall, the right side wall, and the back wall of the pathogen containment chamber.
Fig-11 is an exploded lower left side perspective view of the pathogen containment chamber.
Fig-13 is an exploded view of an entry port cover attachment.
Fig-14 is a perspective view which further illustrates the access port cover attachment.
Fig-15 is a perspective view which illustrates a removable adhesive cover being removed from the access port cover attachment to expose a layer of adhesive.
Fig-16 is an exploded view of the pathogen containment chamber including the entry port cover attachments.
Fig-17 illustrates the fully assembled pathogen containment chamber including the access port cover attachments.

The reference numerals used in the drawings used to identify the elements of the aerosol box of the prior art and the pathogen containment device of this invention are as follows:
1 - pathogen containment device
2 - pathogen containment chamber
3 - left side wall (of the pathogen containment chamber)
4 - right side wall (of the pathogen containment chamber)
5 - back wall (of the pathogen containment chamber)
6 - top (of the pathogen containment chamber)
7 - patient
8 - head of the patient
9 - healthcare professional
10 - hands (of healthcare professional)
12 - access port(s)
13 - flexible sheet
14 - laryngoscope
15 - optional entry port(s)
16 - roll of flexible sheet
17 - air tight removable covering
18. - pathogen containment kit
19 - roll of flexible sheet
20 - air exhaust coupling
21 - air exhaust line
22 - air pump
23 - disinfecting zone
24 - packaging container
25 - wall assembly
26 - access port cover(s)
27 - equipment access port(s)
28 - flexible strips
29 - lip
30 - adhesive surface
31 - access port cover attachment
32 - access port cover support
33 - adhesive tape
34 - removable adhesive cover strip

### Detailed Description of the Invention

Fig-1 and Fig-2 are a perspective view taken from the back side of the pathogen containment device **1** of this invention. As can be seen, the pathogen containment chamber **2** is comprised of a left side wall **3,** and right side wall **4,** a back wall **5,** and a top **6** with two access ports **12** which are adapted for allowing the hands **10** of a health care professional **9** access into the pathogen containment chamber **2.** As can be seen, these access ports **12** are sufficient in size to allow the hands **10** of a healthcare professional **9** access therethrough and also are of sufficient size to permit the degree of movement necessary to carry out the required medical procedure with needed equipment, such as a laryngoscope **14.** Typically, the access ports **12** are essentially circular and have a diameter which is within the range of about 4 inches to about 6 inches and normally have a diameter which is within the range of about 4.5 inches to about 5.5 inches.

Fig-3 illustrates the hands **10** of a health care professional **9** extending into the pathogen containment chamber **2** in a manner whereby the health care professional **9** is capable of carrying out needed procedures on the head **8** of a patient **7.** As can been seen, the access ports **12** on the back wall **5** of the pathogen containment chamber **2** allow sufficient access for the health care professional **9** to manipulate medical instruments, such as a laryngoscope **15,** within the pathogen containment chamber **2** as need to carry out needed medical procedures

In various embodiments of this invention the pathogen containment chamber **2** can be a clear square, rectangular, trapezoid, or circular box with open areas for the hands **10** of a practitioner and/or assistants to access and serve the needs of a patient **7.** These access ports **12** can be customized as required to perform desired tasks. The pathogen containment chamber **2** is typically made of light clear plastic that can optionally be colored or darkened as needed to facilitate the conduction of needed medical procedures. It can also include optional internal and/or external lightening sources, access points for suction of air, or for the delivery of aerosolized treatments. The pathogen containment device **1** can also optionally include flexible extension sheets **13** which are attached to the front, sides and/or back of the pathogen containment chamber **2** and which can be adapted to fully or partly cover the body of the patient **7** to better contain pathogens with the pathogen containment device **1.** In any case, the pathogen containment device can be designed to (1) protect healthcare workers, (2) protect the patient, and/or (3) provide the patient with a beneficial controlled environment.

The pathogen containment device **1** of this invention can be used to protect doctors, dentists, nurses, dental hygienists, health care workers, beauticians, facial cosmetologists, tattoo artists, makeup artists, and others from any harmful elements and pathogens that the patient may emit into the environment (air) around them. It can be of particular value in carrying out medical procedures, such as the introduction or withdrawal of intubation including, but not limited to, endo-tracheal tubes, naso tracheal tubes, LMA tubes, nasogastric tubes, tracheostomy tubes, gastric tubes, tracheostomy care, bronchoscopy, endoscopy, tracheal suction or irrigation, central line, EGD, and the like. The pathogen containment device **1** of this invention is of particular value in conducting such procedures on contagious patients having bacterial, myco-bacterial, viral, or fungal infections. Continued use of the pathogen containment device **1** of this invention after such procedures have been completed through the end of the period during which the patient **7** is contagious can be high desirable in limiting the spread of disease to health care professionals **9** caring for the patient **7.** For example, the pathogen containment device **1** of this invention can be kept on the patient **7** for a period on many days of even longer than a week. In another scenario, the pathogen containment device **1** can be placed over the head **8** of any patient showing signs of infection, such as elevated temperature, labored breathing, gaging, coughing, sneezing, vomiting, or belching, without the need to carry out any type of intubation procedure or other specific procedure.

In some cases the pathogen containment device **1** of this invention is useful for the purpose of isolating a patient **7** from airborne pathogens originating from health care workers or visitors, and/or unhealthy environment conditions, including dust, harmful airborne particles, allergens, perfumes, toxic gases, or light. The use of the device of this invention for reverse isolation of a patient can be particularly beneficial in the case of immune compromised patients, such as those having neutropenia. It can also be useful as a barrier for burn patients and to prevent drying or insensible fluid loss.

In another scenario the pathogen containment device **1** of this invention can be used to provide a patient **7** with a clean filtered air or a beneficial controlled environment, such as temperature control, humidity control, pH control, and oxygen content control. It can also be used in therapy, including light therapy or the application of mist, oxygen fortified air, and/or aerosolized medications under conditions of an engineered and controlled environment.

Fig-4 illustrates the pathogen containment device **1** of this invention with a patient **7** being positioned with a flexible sheet **13** which is attached to the containment chamber **2** partially covering the patient **7** in a manner that impedes pathogens from escaping from the pathogen containment chamber **2.** In this embodiment of the invention a flexible sheet **13** extends from the left side **3,** the right side **4,** and the top **6** of the pathogen containment chamber **2** in a manner which inhibits the flow of contaminated air from the inside to the outside of the pathogen containment device **1.** In any case, the flexible sheet **13** impedes pathogens from escaping from the pathogen containment device **1** and entering into the surrounding environment. The flexible sheet **13** can be permanently attached to the pathogen containment chamber **2** or it can be attached and detached as needed with adhesive tape, a hook and loop fastener, such as Velcro, or some other means. The flexible sheet **13** can be comprised of plastic, a nonwoven fabric, or a woven fabric, such as a woven cotton sheet. However, it is preferable for the material to have a low degree of air permeability.

Fig-5 is a top view of the pathogen containment device **1** showing an optional entry port **15** on the top **6** of the containment chamber **2** and a roll of flexible sheet **16** which is attached to the containment chamber **2** and which can be unrolled so as to be extended partially or totally over a patient **7** in a manner that inhibits pathogens for escaping to the environment on the outside of the pathogen containment device **1.** Optional entry ports **15,** such as the one illustrated in Fig-5, which are positioned on the top side **6** of the containment chamber **2** can preferably be sealed and resealed as needed to treat a patient. This can be done by simply positioning an essentially air tight removable covering **17,** such as a plastic film, over the optional access port **15** which is not being used at the time.

Fig-6 illustrates the pathogen containment device **1** of this invention with a patient **7** being positioned within the device. Fig-6 shows the patient **7** being covered with a flexible roll of sheet **16** so as to reduce the level of pathogen laden air that can escape from the device into the environment surrounding the patient **7.** The flexible sheet

Fig-7 is a side perspective view of the containment chamber **1** showing an air exhaust coupling **20** which extends through right side wall **4** of the pathogen containment chamber **2.** As can be seen an exhaust line **21** is connected to the air exhaust coupling **20** through which contaminated air is pumped from the containment chamber **2** with an air pump **22** and then passed through a disinfecting zone **23** which is capable of destroying pathogens in the contaminated air. This can be accomplished by sparged (bubbling) the contaminated air through a container, such as a bottle of disinfecting solution. For example, the disinfecting solution can be an aqueous sodium hypochlorite solution or an alcohol solution. The contaminated air can also be passed through a zone of which exposes it to a sufficient level of ionizing radiation to destroy or eradicate any pathogens that may be present. For instance, the air can be passed through an area that exposes it to a high level of ultra-violet light or an electron beam. The contaminated air can also be passed through a high efficiency filter which is capable of trapping bacteria and virus. For example the filter can be a one-gallon ULPA filter which is comprised of 56 pleat media including one cellulose layer and two glass layers which have a 99.999% efficient of trapping 0.12 micron particles including virus.

In one embodiment of this invention, contaminated air from the pathogen containment chamber **2** is drawn through the exhaust line **21** and into the vacuum system of a building, such as a hospital, clinic, or other health care facility, in which the pathogen containment device **1** is being used. In this particular embodiment of the invention the air exhaust line **21** is connected to a standard hospital suction (vacuum) coupling. The air exhaust line **21** is connected to the pathogen containment chamber **2** through the air exhaust coupling **20** to create a negative pressure within the pathogen containment chamber **2** thereby helping to prevent pathogens from escaping into the atmosphere surrounding the pathogen containment device **1.** The level of suction applied will normally be sufficient to provide a gas flow rate which is within the range of 20 liters per minute to 4000 liters per minute and will typically be within the range of 30 liters per minute to 3000 liters per minute. It is, of course, important for the contaminated art being exhausting to be released into an area where it does not pose a health risk. In cases where the contaminated air is reintroduced into the room where the patient is being treated or where other people may be present it should be passed through a disinfecting zone, such as a disinfecting solution, ionizing radiation, or a ULPA filter, as previously explained.

In a preferred embodiment of this invention the pathogen containment chamber is supplied in a compact form wherein the sides of the chamber have been folded together into a relatively thin square or rectangular shaped piece. In one embodiment of this invention the pathogen containment device **1** is provided in a pathogen containment kit **18.** By virtue of their compacted size and shape these compacted pathogen containment chambers can be sealed in plastic bags to keep them clean for subsequent use after shipment and storage. It is preferable for them to be sealed in resealable plastic bags, such as zip-lock bags, so that they can be again deposited into such bags for safe disposal (for instance in hospital waste) in their folded state. In this embodiment of the invention the top side **6** of the compacted pathogen containment chamber is be folded over the back section **5** with the left side **3** being folded under the back section **5** and the right side **4** being folded under the lift side **3.** It is, of course, possible for the various sides of the pathogen containment chamber to be folded over or under the back section **5** in other orders. For instance, the top side **6** could be folded over the back section **5** with the left side **3** being folded under the back section **5** and the right side **4** being folded under the lift side **3.** In still another scenario, the top side **6,** the left side **3,** and the right side **4** could all be folded under the back section **5** in any desired order.

After being unfolded the assembled pathogen containment chamber **2** can be used in the intubation of patients for any number of other procedures that are being performed on infectious patients. The walls will preferably include a means for holding them together in the assembled position such as snap locks, ties, or adhesive tape. In a preferred embodiment of this invention the assembled pathogen containment chamber **2** includes two patient access ports **12** on its back wall **5,** an optional access port **15** on its right side wall **4,** and one or more optional air exhaust line ports or air exhaust couplings **20** on either its right side wall **4** or its left side wall **3.** In one embodiment of the invention the pathogen containment chamber **2** is about 20 inches wide (0.51 meter), about 16 inches deep (0.41 meter), and has a height of about 19 inches (0.48 meter). However, the width, depth, and height of the assembled pathogen containment chamber can vary with the width typically being within the range of 18 inches (0.46 meter) to 24 inches (0.61 meter), the depth typically being within the range of 15 inches (0.38 meter) to 22 inches (0.56 meter), and the height being within the range of 18 inches (0.46 meter) to 22 inches (0.56 meter).

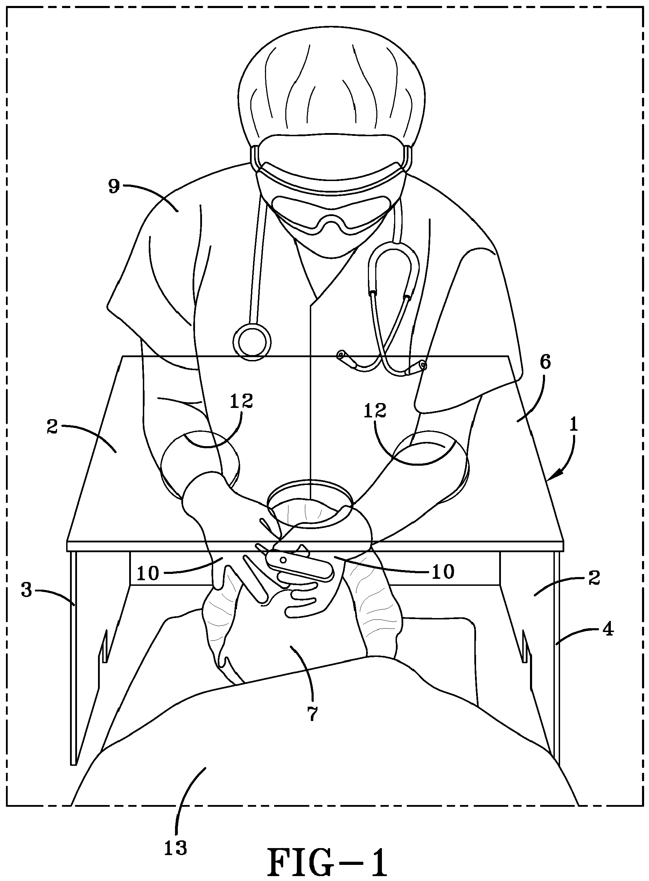

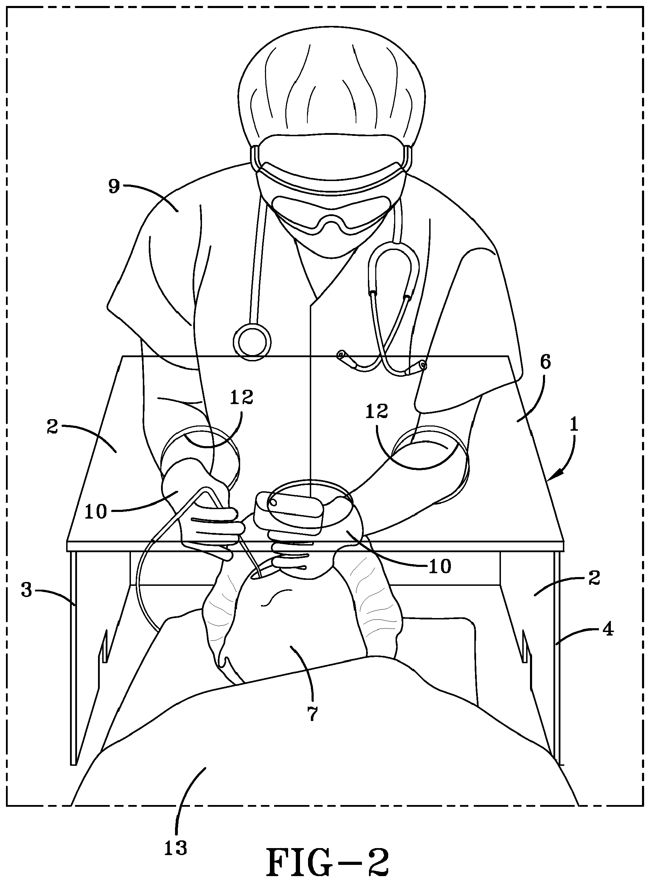

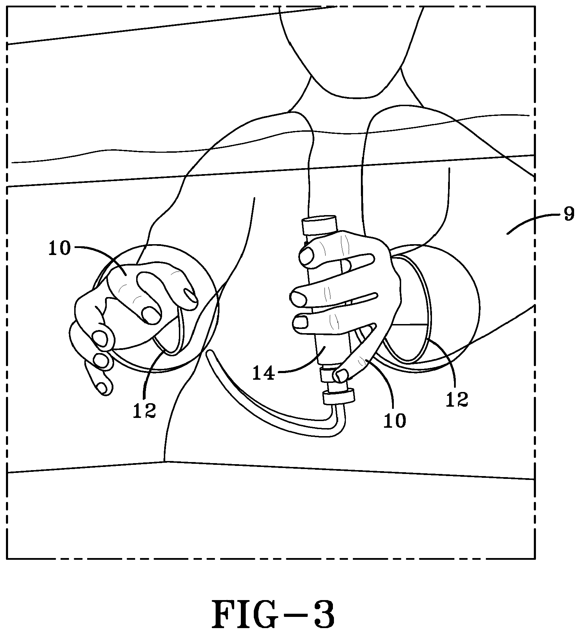

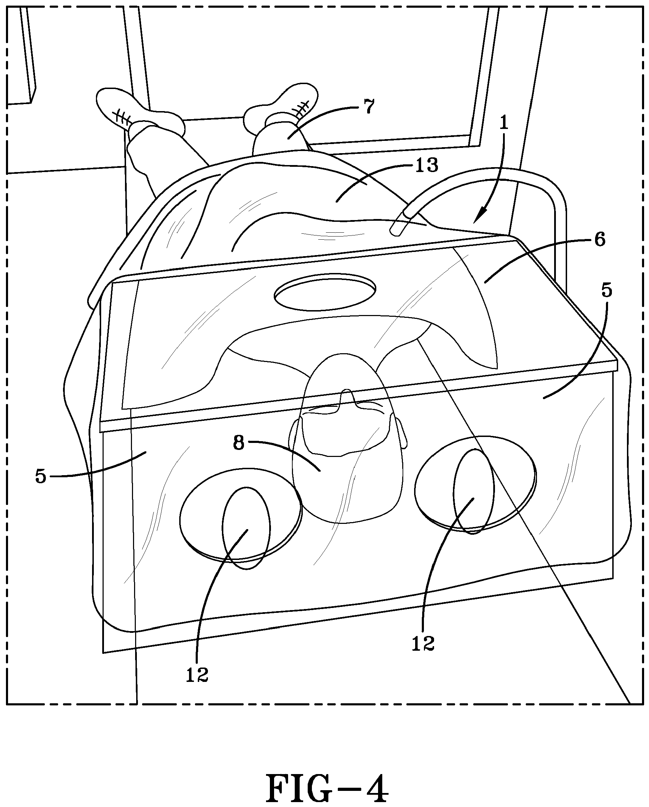

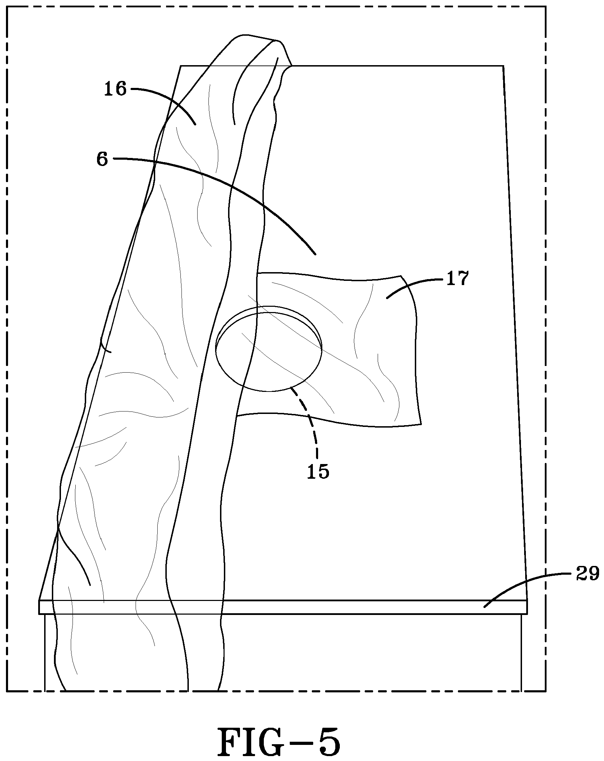

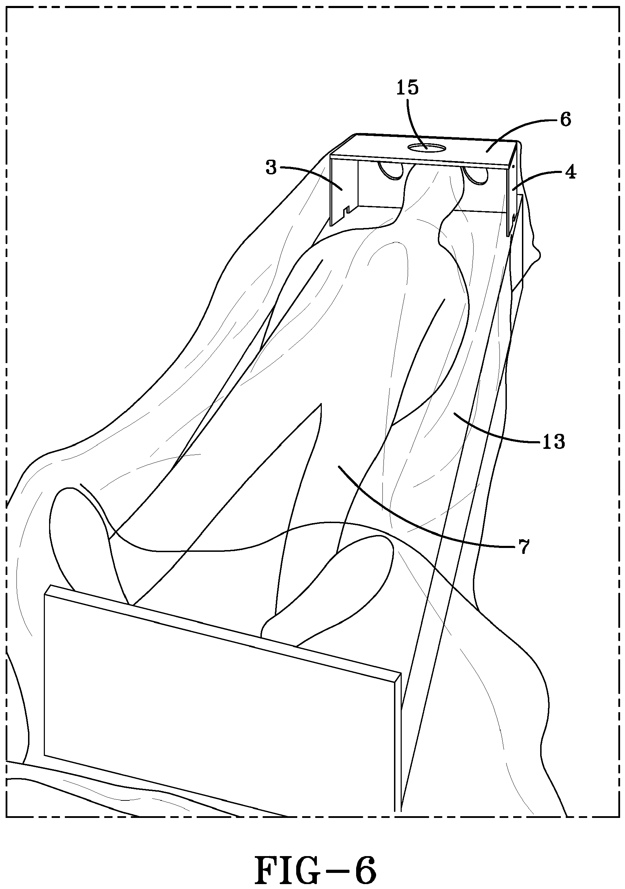

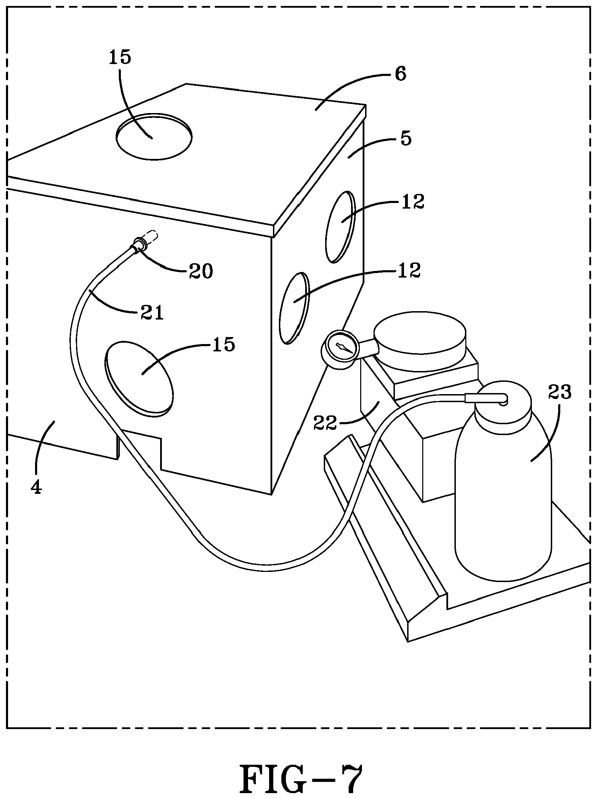

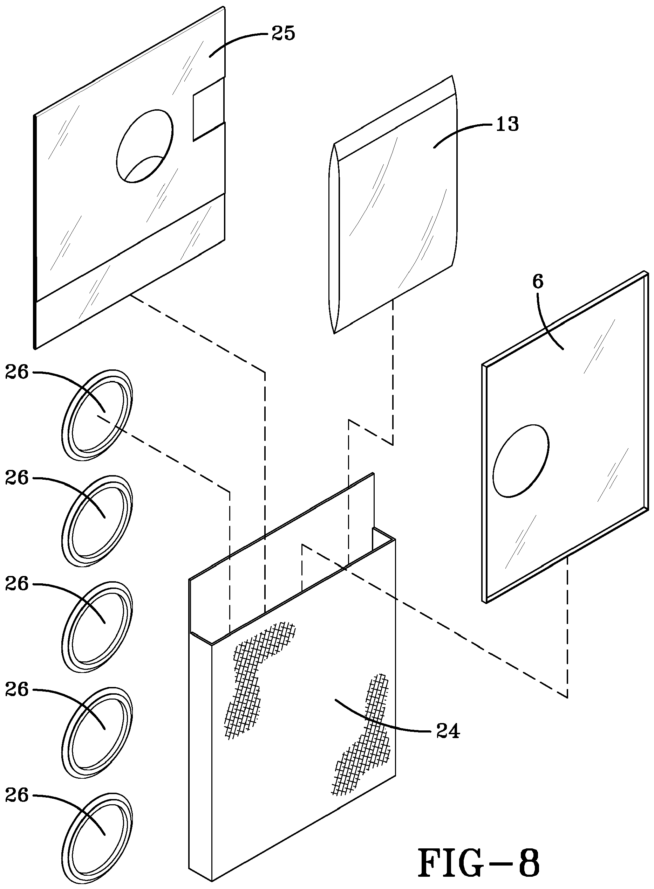

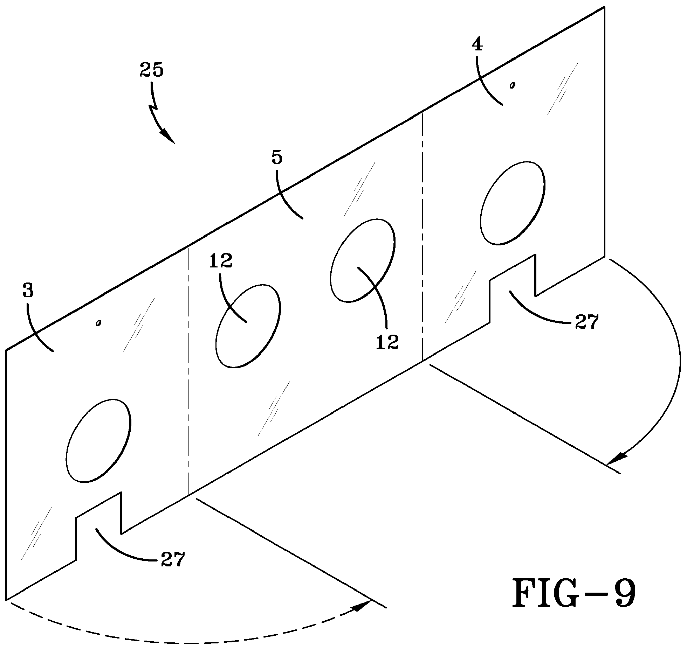

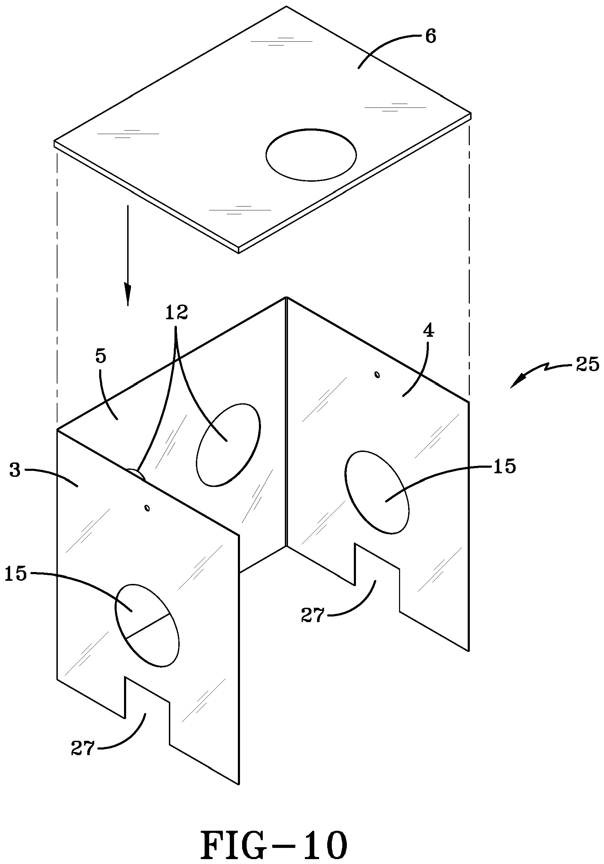

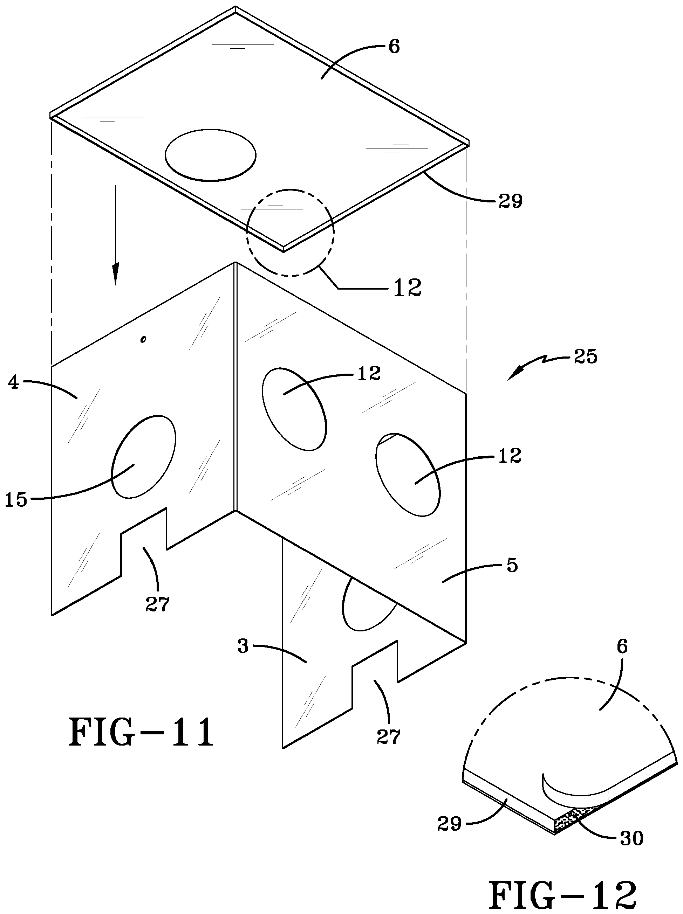

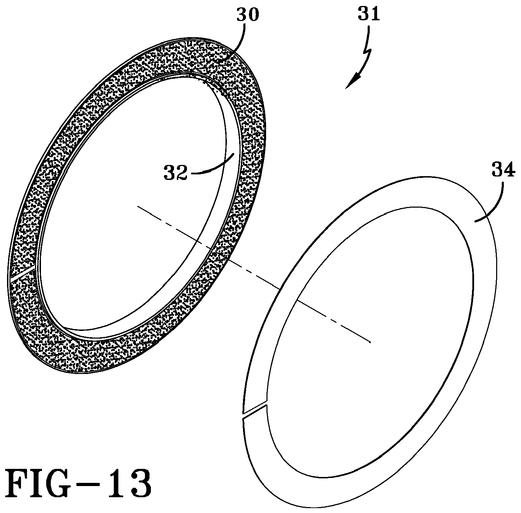

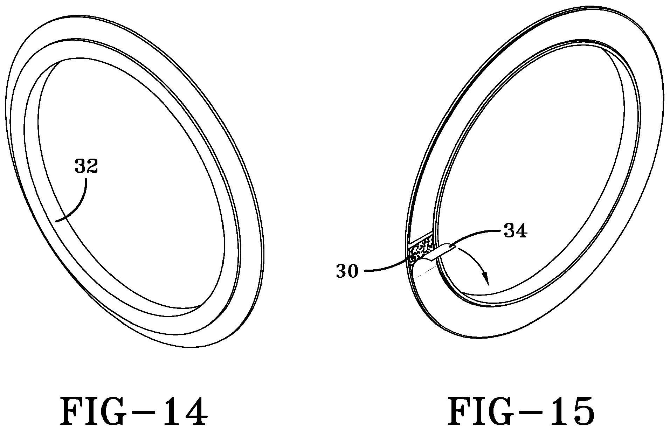

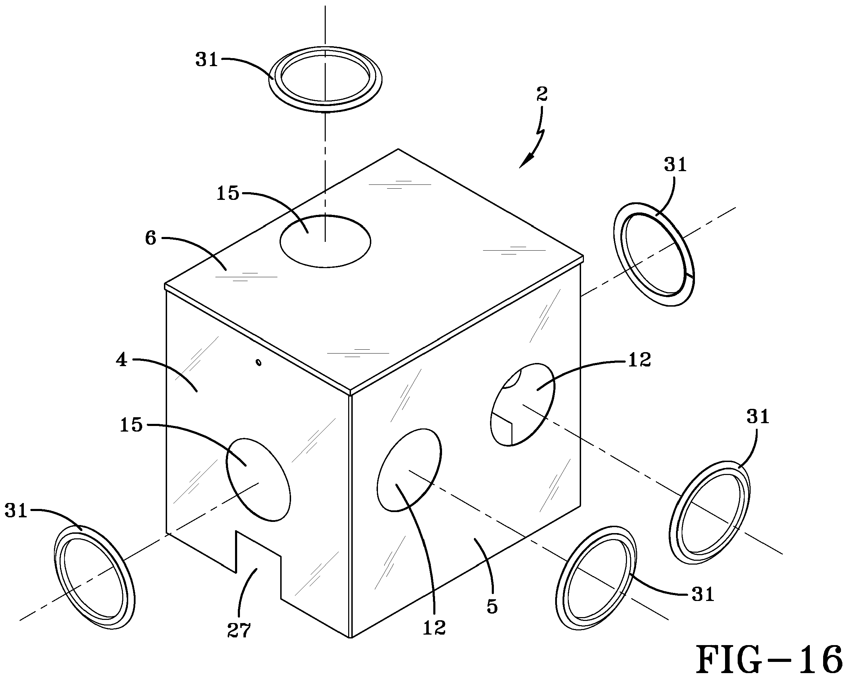

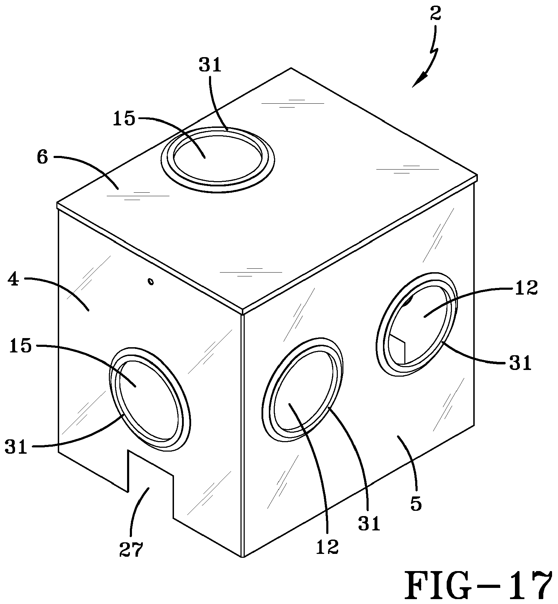

Figure 8 is an exploded view of a pathogen containment kit **18** of this invention. The pathogen containment kit **18** is comprised of a packaging container **24,** a wall assembly **25** (which is comprised of the left side wall **3,** the right side wall **4,** and the back wall **5**), a top **6,** optionally a flexible sheet **13** which is adapted for attachment to the assembled pathogen containment chamber **2,** and optionally a plurality of access port covers 26. The packaging container **24** can be a box, a bag, such as a zip lock bag, or another container that is capable of conveniently holding the components of the pathogen containment device **1.**

Fig-9 illustrates the wall assembly **25** with the left side wall **3** and the right side wall **4** being fully extended from the back wall **5.** As can be seen, the back wall **5** includes two access ports **12** are positional apart horizontally to conveniently allow both hands of a health care professional to extend into the fully assembled pathogen containment chamber **2.** The left side wall **3** and the right side wall **4** both include optional access ports **15** in this embodiment of the invention. In this embodiment of the invention the left side wall **3** and the right side wall **4** both include optional equipment access ports **27** through which equipment and/or intubation tubes can pass into the pathogen containment chamber **2.** In any case the optional access ports **15** and optional equipment access ports **27** can be covered with air tight coverings to inhibit the escape of pathogens from the pathogen containment device **1.**

Fig-10 is an exploded view showing the left side wall **3** and the right side wall **4** extending from the back wall **5** at 90° angles which allows the top **6** to be lowered firmly onto the wall assembly **25** so as to make an essentially air tight seal between the left side wall **3,** the right side wall **4,** and the back wall **5** of the pathogen containment chamber **2.** The left side wall **3** and the right side wall **4** are flexibly attached to the back wall **5** with flexible strips **28.** The flexible strips **28** can be comprised of a flexible adhesive tape or preferably a thin layer of plastic. For instance, the wall assembly **25** of the pathogen containment chamber **2** can be made in a single molding wherein the left side wall **3** and the right side wall **4** are separated from the back wall **5** by flexible strips **28** with the flexible strips **28** being thinner than the walls to facilitate the ease with which the sides can be folded and unfolded from the back wall **5.** These flexible strips act as living hinges through which the left side wall **3** and right side wall **4** are connected to the back side wall **5.** It should be noted that such a living hinge is thin and flexible enough to allow movement between two otherwise rigid sections of the same part (the left side wall **3,** the right side wall **4** and back wall **5** of the pathogen containment chamber). This wall assembly **25** is designed in such a fashion that all three sections (the left side wall **3,** the right side wall **4,** back wall **5,** and the living hinges) are fabricated in one single form. The living hinge strip is designed to allow rotational movement of at least 90° and preferably at least 180°. These flexible strips will typically have a thickness which is within the range of 4 mils (0.10 mm) to 12 mils (0.30 mm), will more typically have a thickness which is within the range of 6 mils (0.15 mm) 10 mils (0.25 mm), and will preferably have a thickness which is within the range of 7 mils (0.18 mm) to 9 mils (0.23 mm). The sides and top of the pathogen containment chamber will typically have a thickness which is within the range of 14 mils (0.36 mm) to 100 mils (2.54 mm), will more typically have a thickness which is within the range of 16 mils (0.41 mm) to 50 mils (1.27 mm), and will preferably have a thickness which is within the range of 20 mils (0.51 mm) to 40 mils (1.02 mm).

The plastic used in manufacturing the pathogen containment chamber should be clear for healthcare workers to have an excellent view of the patient. It should also be tough and durable. In cases where a one piece pathogen containment chamber is being made by molding the plastic it should also have enough flexibility to allow for the walls to be folded, such as through living hinges. It has been determined that polyethylene bottle resin having an intrinsic viscosity which is within the range of about 0.72 dl/g to about 0.82 dl/g as measured in a 60:40 phenol:tetrachloroethane solvent system at a temperature of 30°C and at a concentration of 0.4 g/dl has excellent characteristics for this purpose. Polyethylene terephthalate having these characteristics is commercially available from DKA Americas as Cleartuf® 8600 polyester resin having an IV of 0.80 dl/g.

Fig-11 is an exploded lower left side perspective view of the pathogen containment chamber **2.** As can be seen the top **6** includes lips **29** that extend downwardly so that an essentially air tight seal is made when the top **6** is lowered onto the wall assembly **25.** The top **6** can optionally be taped onto the wall assembly **25** to make an even tighter seal and to further stabilize the structural integrity of the assembled pathogen containment chamber **2.** Fig-12 is an expanded view of area 12 of the top **6** which more clearly shows the lip **29** and optional adhesive tape **33** that can be used to further secure the top **6** to the left side wall **3,** the right side wall **4,** and the back wall **5** of the pathogen containment chamber **2.**

Fig-13 is an exploded view of an entry port cover attachment **31** that can optionally be used to conveniently affix a cover over access ports. The entry port cover attachment **31** is comprised of an access port cover support **32** which is adapted to firmly fit into the access port. The access port cover support **32** will preferably have an adhesive surface **30** covering the top surface thereof and a removable protective cover **34** which covers the adhesive surface **30** until such time that it is desired to cover the access port by affixing a film or fabric to the exposed adhesive on the entry port cover attachment **31.** Fig-14 and Fig-15 further illustrate the access port cover attachment. Fig-16 is an exploded view of the pathogen containment chamber **2** including the entry port cover attachments **31** and Fig-17 illustrates the fully assembled pathogen containment chamber **2** including the access port cover attachments **31.**

While certain representative embodiments and details have been shown for the purpose of illustrating the subject invention, it will be apparent to those skilled in this art that various changes and modifications can be made therein without departing from the scope of the subject invention.

## Claims

1. A pathogen containment device which is particularly useful for intubating human patients, said device being **characterized by** including a pathogen containment chamber which is adapted for being positioned over and covering the head of the patient, said containment chamber having two access ports which are adapted for allowing the hands of a health care worker access into the containment chamber, and said containment chamber having an air exhaust coupling, wherein the containment chamber is at least partially comprised of an essentially transparent material which allows for the health care worker to have a sufficient view of the head of the patient to perform intubation of the patient.

2. The pathogen containment device as specified in claim 1 which is **characterized in that** the containment chamber is comprised of folding sides which are affixed together through living hinges.

3. The pathogen containment device as specified in claims 1 or 2 which is **characterized in that** the pathogen containment chamber is comprised of a wall assembly and a top, wherein the wall assembly includes a back, a left side, and a right side, wherein the left side and the right side are capable of being folded onto the back, and wherein the left side and the right side are capable of being extended to be perpendicular to the back, and wherein the top is configured to fit over the left side, the back, and the right side in an essentially air tight manner when the left side and the right side are in a configuration where they are extended to be perpendicular to the back.

4. The pathogen containment device as specified in claim 3 which is **characterized in that** the right side wall and the left side wall are connected to the back wall through living hinges.

5. The pathogen containment device as specified in claim 3 or 4 which is **characterized in that** the top includes lips which are adapted to fit firmly over the left side, the back, and the right side when the left side and the right side are in a configuration where they are extended to be perpendicular to the back.

6. The pathogen containment device as specified in any of the preceding claims which is **characterized in that** the pathogen containment chamber includes an air exhaust coupling.

7. The pathogen containment device as specified in any of the preceding claims which is **characterized by** further including a flexible sheet which is attached to the containment chamber and which is adapted to cover at least part of the patient in a manner that impedes pathogens from escaping from the containment chamber by sealing the patient within the interior of the pathogen containment device.

8. The pathogen containment device as specified in any of claims 4-7 which is **characterized in that** the pathogen containment chamber is comprised of polyethylene terephthalate having an intrinsic viscosity which is within the range of about 0.72 dl/g to about 0.82 dl/g as measured in a 60:40 phenol:tetrachloroethane solvent system at a temperature of 30°C and at a concentration of 0.4 g/dl, wherein the left side wall, the right side wall, the back wall, and the top of the pathogen containment chamber have a wall thickness which is within the range of 0.36 mm to 2.5 mm, and wherein the living hinges are within the range of 0.10 mm to 0.30 mils thick.

9. The pathogen containment device as specified in claim 8 which is **characterized in that** the left side wall, the right side wall, the back wall, and the top of the pathogen containment chamber have a wall thickness which is within the range of 0.51 mm to 1.02 mils and wherein the living hinges are within the range of 0.18 mm to 0.23 mm thick.

10. A pathogen containment kit which is **characterized by** including the pathogen containment device as specified in any of claims 1-9 wherein the sides of the containment chamber are folded together and enclosed within a container.

11. The pathogen containment kit as specified in claim 10 which is **characterized in that** the container is a resealable bag.

12. The pathogen containment kit as specified in claim 10 which is **characterized in that** the container is a cardboard box.

13. The pathogen containment kit as specified in claim 10, 11, or 12 which is **characterized by** further including of a flexible sheet which is adapted for attachment to the pathogen containment chamber in an essentially air tight manner and which is adapted for at least partially covering the body of a patient.

14. The pathogen containment kit as specified in claim 10, 11, 12, or 13 which is **characterized by** further including at least one access port cover attachment.

15. A method for intubating a patient which comprises (1) positioning the patient in an essentially horizontal position for performing the intubation; (2) positioning the pathogen containment device as specified in claim 7 over the head and at least a portion of the body of the patient; (3) activating the means for exhausting air from the containment chamber; and (4) intubating the patient.
